# EUROPEAN PATENT SPECIFICATION

(11) **EP 1 767 246 B1**
(45) Date of publication and mention of the grant of the patent: **13.08.2008**
(21) Application number: 06020163.9
(22) Date of filing: 26.09.2006
(51) Int. Cl.: A61P 1/02, A61K 31/765, A61K 31/047

(54) **Composition for moistening oral cavity**
Zusammensetzung zur Befeuchtung der Mundhöhle
Composition pour l'hydratation de la cavité buccale

(30) Priority: 26.09.2005 JP 2005277587
(43) Date of publication of application: 28.03.2007
(73) Proprietor: GC Corporation, Itabashi-ku, Tokyo (JP)
(72) Inventor: Sato, Takuya, Tokyo (JP); Chen, Li, Tokyo (JP)
(74) Representative: Müller-Boré & Partner Patentanwälte

(56) References cited:
- EP-A1- 1 566 166
- WO-A-99/22703
- US-A- 5 541 165
- DATABASE WPI Week 198905 Derwent Publications Ltd., London, GB; AN 1989-037180 XP002413400 -& JP 63 311960 A (SEKISUI CHEM IND CO LTD) 20 December 1988 (1988-12-20)

## Description

The present invention relates to a composition for moistening an oral cavity, which is used for maintaining moisture on an oral mucosa surface.

A human oral mucosa is generally moistened with saliva, but a feeling of dryness occurs when the secretion of saliva is lowered. There is such a tendency that the secretion amount of saliva is lowered with advancing age due to atrophia of the salivary gland, and a case causing strong feeling of dryness in the oral cavity is referred to as xerostomia (dry mouth). Xerostomia also occurs due to disease of salivary gland (such as chronic inflammation of salivary gland, Sjogren syndrome and Mikuliz syndrome), disease causing damage of nerve participating in secretion nerve center or secretion, diabetes, and exposure to radiation of an oral cavity, a nasal cavity or a throat, in addition to physiological decrease in saliva secretion amount with advancing age. Advance of xerostomia causes dryness on glossal mucosa and labial mucosa to induce fissures to easily cause bleeding, and brings about such problems as disability not only in manducation and deglutition but also in degustation, disability in speech, and difficulty in retention of denture.

In order to eliminate the problems, artificial saliva has been developed. Examples of the artificial saliva include artificial saliva containing an inorganic salt with mucin derived from mammals other than human (as described, for example, in JP-A-57-500562), artificial saliva containing hydroxypropyl cellulose, methyl cellulose or hydroxypropylmethyl cellulose as a thickening agent, and sodium guaiazulene sulfonate as a bactericide (as described, for example, in JP-A-59-007116 and JP-A-59-027818), artificial saliva containing a carboxymethyl cellulose alkali salt having an etherification degree of 150 or more as a thickening agent (as described, for example, in JP-A-61-151118), and artificial saliva containing a polymer providing an aqueous solution having spinnability, such as polyacrylic acid, as a thickening agent, and a phosphate buffer (as described, for example, in JP-B-55-026121).

However, the artificial saliva that is only increased in viscosity with a thickening agent can only provide an effect of extending the retention time of the composition in an oral cavity, but cannot control moisture on an oral mucosa surface.

In order to control moisture on an oral mucosa surface positively, artificial mucus containing sorbitol, xylitol, glycerin or polyglycerin as a moisturizing agent (as described, for example, in JP-A-62-236862), and an oral moistening agent for denture containing a specific water soluble polymer, a specific polyhydric alcohol, and water and/or artificial saliva and exhibiting adherence through swelling or gelation with water (as described, for example, in JP-A-2004-136102) have been developed.

The artificial mucus and the oral moistening agent for artificial denture contain a moisturizing component, for example, 1-5% by weight of a moisturizing agent is contained in the oral moistening agent in JP-A-2004-136102. However, they have such a problem that moisture contained in the composition is effused to a dry oral cavity due to the small content of the moisturizing component, so as to fail to retain sufficiently the effect of moisturizing the oral mucosa.

In the case where the content of the moisturizing component is increased to eliminate the problem, another problem occurs in that upon applying the composition to an oral mucosa of a patient with xerostomia, the composition absorbs moisture of the oral mucosa with less moisture.

Accordingly, in order to cope with xerostomia, it is important that not only the composition contains a thickening agent to be retained in an oral cavity for a prolonged period of time, but also the amount of moisture contained in the composition effused to an oral cavity is small (hereinafter, sometimes referred to as low moisture effusion property), and simultaneously the composition is hard to absorb moisture of an oral mucosa (hereinafter, sometimes referred to as low moisture absorption property).

An object of the invention is to provide such a composition for moistening an oral cavity that has a small effusion amount of moisture contained in the composition itself, is hard to absorb moisture of an oral mucosa, and is capable of continuously supplying moisture stably to an oral mucosa surface.

As a result of earnest investigations made by the inventors for solving the problems, it has been found that a composition for moistening an oral mucosa having excellent water retaining property can be obtained by using polyglycerin and a particular water soluble polymer in combination.

The invention relates to a composition for moistening an oral cavity containing 2-10% by weight of glycerin, 25-60% by weight of polyglycerin, 20-60% by weight of water, and 0.1-15% by weight of at least one water soluble polymer as a thickening agent selected from the group consisting of sodium alginate, gum arabic, agar, traganth gum, carrageenan and xanthan gum.

The composition for moistening an oral cavity according to the present invention is an excellent composition for moistening an oral cavity that has a small effusion amount of moisture contained in the composition, and is hard to absorb moisture of an oral mucosa.

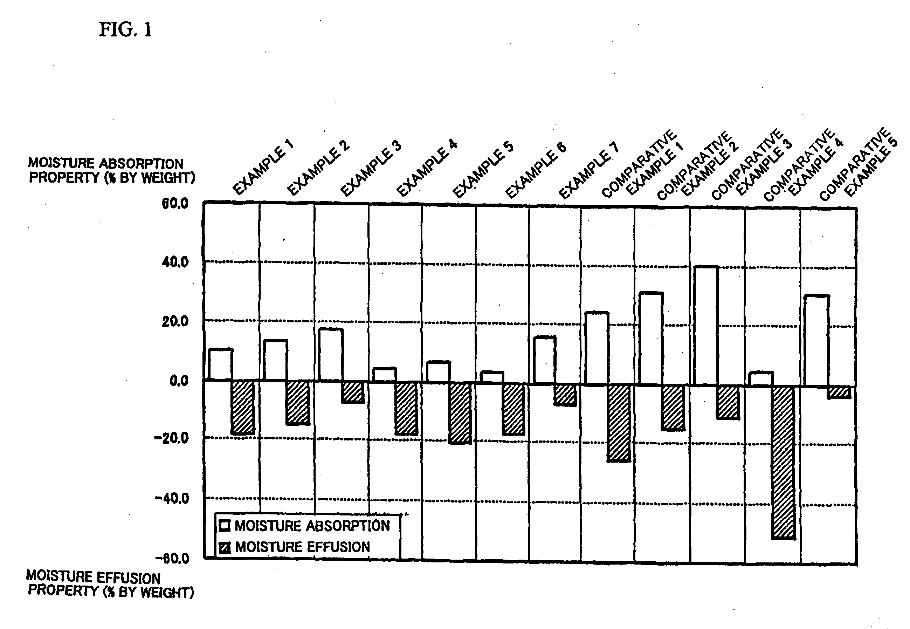

Fig. 1 is a diagram showing results of evaluation of moisture absorption property and moisture effusion property of the examples and the comparative examples.

The composition for moistening an oral cavity according to the present invention contains, as a moisturizing component, 2-10% by weight of glycerin and from 25 to 60% by weight of polyglycerin, which may be diglycerin or higher polyglycerin and may have an average number of glycerin units of 20 or less. The average number of glycerin units of the polyglycerin ispreferably2-7. In the case where the average number of glycerin units exceeds 7, there is such a tendency that the stability of the effect of suppressing moisture effusion and moisture absorption of the composition is lowered. The composition for moistening an oral cavity according to the present invention contains less glycerin than polyglycerin because of the following factors. In the case where the moisture effusion property of the composition to an oral cavity is suppressed by using a large amount of glycerin, the moisture absorption property of the composition from an oral cavity is increased. In the case where the moisture absorption property is suppressed, on the other hand, the moisture effusion property is drastically increased. Furthermore, in the case where the moisture effusion property and the moisture absorption property of the composition are controlled to be equal to each other only with glycerin, both the properties become too high, which are not suitable as a composition for moistening an oral cavity. Moreover, the amount of glycerin exceeding 10% by weight is not favorable from the standpoint of degustation due to the strong sweetness thereof.

As described earlier, the case where glycerin is used in a large amount as a moisturizing agent of a composition for moistening an oral cavity, the balance between the moisture effusion property and the moisture absorption property of the composition is difficult for control. Accordingly, use of glycerin in the composition for moistening an oral cavity of the present invention is not preferable, but commercially available polyglycerin contains a certain amount of free glycerin unless otherwise purified, and the composition for moistening an oral cavity of the present invention substantially contains 2-10% by weight of glycerin.

In the case where polyglycerin is used in combination with the particular water soluble polymer described later, the moisture effusion property and the moisture absorption property of the composition can be brought approximate to those in an oral cavity. Furthermore, polyglycerin is weak in sweetness as compared to glycerin and thus is favorable in use as in the composition for moistening an oral cavity. Taking the high viscosity of polyglycerin as compared to glycerin into consideration, polyglycerin can provide such a composition for moistening an oral cavity that has less sweetness and good feeling in use.

The mixing amount of polyglycerin in the composition for moistening an oral cavity of the present invention is 25-60% by weight. In the case where the amount of polyglycerin is less than 25% by weight, the moisture effusion property of the composition for moistening an oral cavity becomes too high irrespective of other components. In the case where the amount thereof exceeds 60% by weight, the moisture absorption property of the composition becomes too high irrespective of other components, and furthermore the sweetness of the composition is increased to impair the feeling in use.

The composition for moistening an oral cavity of the present invention contains 20-60% by weight of water. In the case where the amount of water is less than 20% by weight or exceeds 60% by weight, the moisture effusion property and the moisture absorption property of the composition for moistening an oral cavity cannot be brought approximate to those in an oral cavity.

The particular water soluble polymer contained in the composition for moistening an oral cavity of the present invention can exert the effect thereof by containing at least one selected from the group consisting of sodium alginate, gum arabic, agar, traganth gum, carrageenan and xanthan gum. Among these, carrageenan is preferable from the standpoint of swelling, gelation capability, and adherence to an oral mucosa. The thickness agent of the particular water soluble polymer is used in combination with polyglycerin, whereby the moisture effusion property and the moisture absorption property of the composition can be optimally controlled and brought approximate to properties in an oral cavity.

The particular water soluble polymer as a thickening agent may be used in combination with an ordinary water soluble polymer having been used as a thickening agent, examples of which include a natural polymer, such as pullulan and dextran, a semi-synthetic polymer, such as dextrin, methyl cellulose, carboxymethyl cellulose, sodium carboxymethyl cellulose and hydroxyethyl cellulose, and a synthetic polymer, such as polyvinylpyrrolidone, carboxyvinyl polymer, polyvinyl alcohol andmacrogol. The ordinary water soluble polymer is preferably used in an amount less than the amount of the particular water soluble polymer.

The thickening agent of water soluble polymer base containing the particular water soluble polymer is contained in the composition for moistening an oral cavity in an amount 0.1-15% by weight. In the case where the amount thereof is less than 0.1% by weight, the composition cannot have a sufficient viscosity, and in the case where the amount exceeds 15% by weight, the viscosity of the composition becomes too high.

The composition for moistening an oral cavity according to the present invention contains the aforementioned substances as essential components, but may contain other various additives, such as antiseptic, a colorant, a perfume and a saliva secretion accelerator.

The invention will be described in more detail with reference to examples below, but the present invention is not construed as being limited thereto.

Components were mixed according to the formulations (in terms percent by weight) shown in Table 1 below to prepare compositions for moistening an oral cavity of Examples 1 to 7 and Comparative Example 1 to 3. As polyglycerin, Diglycerin 801, Polyglycerin #310, #500 and #700, all produced by Sakamoto Yakuhin Kogyo Co. , Ltd. , were used. Glycerin as the component was that contained in the polyglycerin and was not added separately. The amount of water was the total amount of water added and water contained in glycerin and polyglycerin.

0.4 g of each of compositions for moistening an oral cavity of Examples 1 to 7 and Comparative Example 1 to 5 was placed on a weighing dish and weighed after allowing to stand under conditions of a humidity of 100% and a temperature of 37°C for 2 hours. A value obtained by dividing the weight change by the initial weight (0.4 g) in terms of percentage was designated as moisture absorption property. The results obtained are shown in Table 1.

0.4 g of each of compositions for moistening an oral cavity of Examples 1 to 7 and Comparative Example 1 to 3 was placed on a weighing dish and weighed after allowing to stand under conditions of a humidity of 0% and a temperature of 37°C for 2 hours. A value obtained by dividing the weight change by the initial weight (0.4 g) in terms of percentage was designated as moisture absorption property. The results obtained are shown in Table 1.

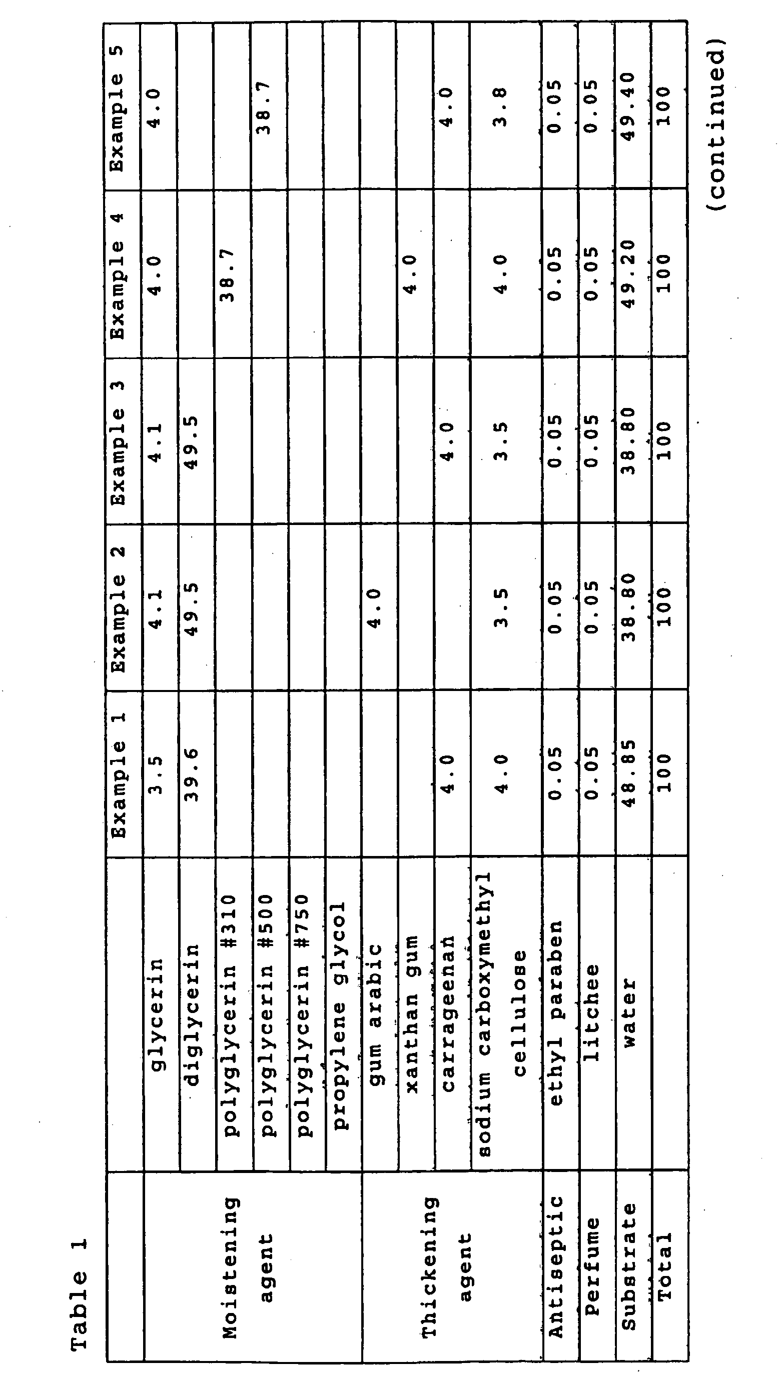

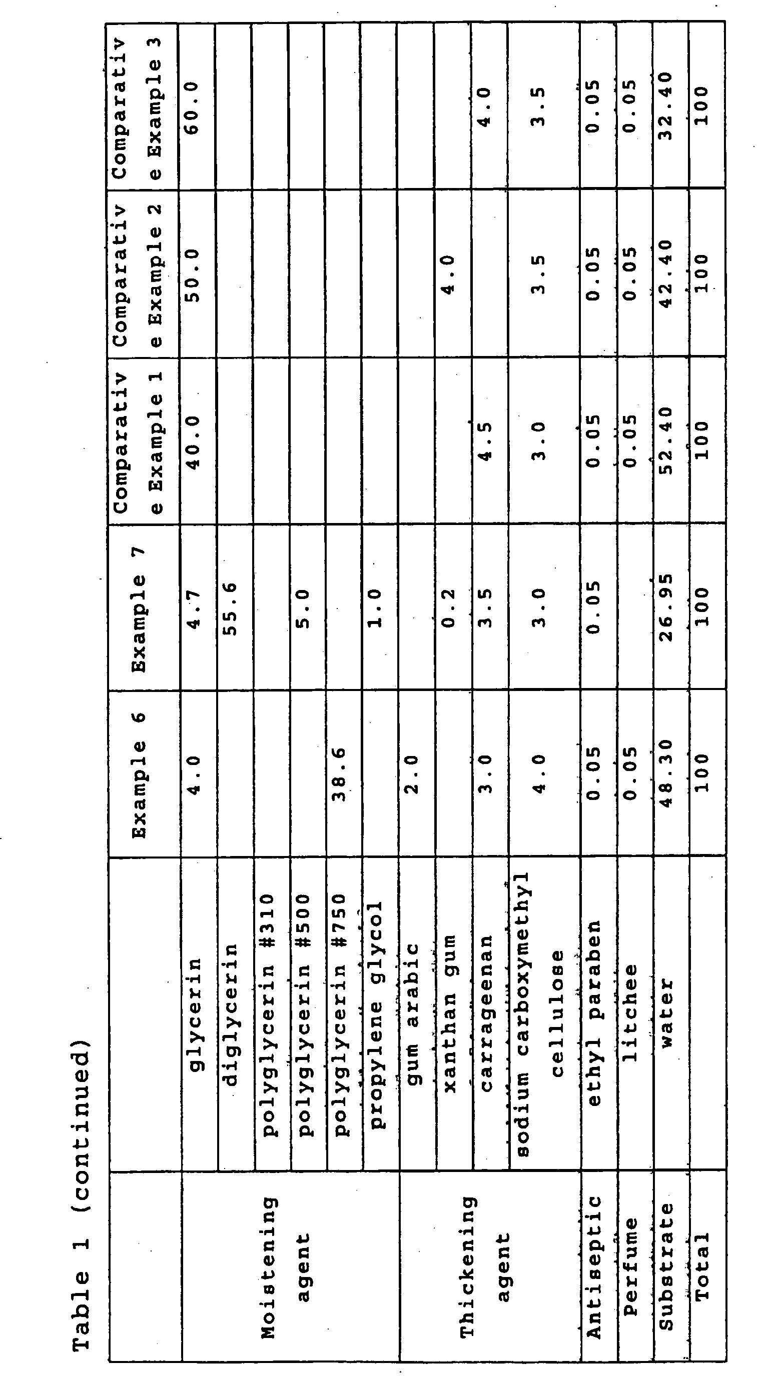

## Claims

1. A composition for moistening an oral cavity comprising 2-10% by weight of glycerin, 25-60% by weight of polyglycerin, from 20-60% by weight of water, and 0.1-15% by weight of at least one water soluble polymer as a thickening agent selected from the group consisting of sodium alginate, gum arabic, agar, traganth gum, carrageenan and xanthan gum.

## Patentansprüche

1. Zusammensetzung zur Befeuchtung einer Mundhöhle, umfassend 2-10 Gew.-% Glycerin, 25-60 Gew.-% Polyglycerin, von 20-60 Gew.-% Wasser und 0,1-15 Gew.-% von mindestens einem wasserlöslichen Polymer als ein Verdickungsmittel, ausgewählt aus der Gruppe, bestehend aus Natriumalginat, Gummiarabikum, Agar, Tragantgummi, Karrageen und Xanthangummi.

## Revendications

1. Composition pour humidifier une cavité orale comprenant 2 à 10 % en poids de glycérol, 25 à 60 % en poids de polyglycérol, 20 à 60 % en poids d'eau et 0,1 à 15 % en poids d'au moins un polymère soluble dans l'eau comme agent épaississant choisi dans le groupe constitué de l'alginate de sodium, de la gomme arabique, de l'agar, de la gomme tragacanthe, de la carraghénine et de la gomme xanthane.
